# EUROPEAN PATENT SPECIFICATION

(11) **EP 1 082 965 B1**
(45) Date of publication and mention of the grant of the patent: **10.06.2009**
(21) Application number: 00203874.3
(22) Date of filing: 19.06.1996
(51) Int. Cl.: A61K 39/39, A61K 39/145

(54) **A vaccine composition comprising a polysaccharide conjugate antigen adsorbed onto aluminium phosphate**
Impfstoff bestehend aus auf Aluminium-Phosphat adsorbiertem, konjugiertem Polysaccharid Antigen
Vaccin comprenant comme antigène un polysaccharide conjugé, adsorbé sur du phosphate d'aluminium

(30) Priority: 23.06.1995 GB 9512827; 01.07.1995 GB 9513443; 15.12.1995 GB 9525657; 22.03.1996 GB 9606032
(43) Date of publication of application: 14.03.2001
(62) Divisional of application: 96922871.7
(73) Proprietor: GlaxoSmithKline Biologicals s.a., 1330 Rixensart (BE)
(72) Inventor: Peetermans, Julien, 1330 Rixensart (BE); Hauser, Pierre, 1330 Rixensart (BE)
(74) Representative: Privett, Kathryn Louise

(56) References cited:
- EP-A- 0 594 950
- BIXLER, JR., G.S. AND S. PILLAI: "Augmentation by interleukins of the antibody response to a conjugate vaccine against Haemopholus influenza B" ADV. EXP. MED. BIOL., vol. 303, 1991, pages 185-190, XP000570343 IMMUNOBIOLOGY OF PROTEINS AND PEPTIDES VI, ED.: M.Z. ATASSI, PLENUM PRESS, NEW YORK, 1991
- CLAESSON, B.A. ET AL: "Clinical and immunologic responses to the capsular polysaccharide of Haemophilus influenzae type b alone or conjugated to tetanus toxoid in 18- to 23-month-old children" THE JOURNAL OF PEDIATRICS, vol. 112, 1988, pages 695-702, XP000604106
- SILBER G.R. ET AL VACCINE vol. 13, no. 6, 1995, pages 525 - 531

## Description

The present invention relates to new vaccine formulations, comprising a conjugated polysaccharide antigen linked to a carrier protein. The invention relates to a vaccine formulation for the prevention of *Heamophilus* Influenzae Type B (Hib) infections and where the antigen is adsorbed on to aluminium phosphate. The invention also relates to a multivalent vaccine, that is a vaccine for the amelioration or treatment of more than one disease states. The present invention also relates to the production and use of such vaccines in medicine.

Vaccines that utilise polysaccharides are known in the art. For example a vaccine for the prevention of Haemophilus influenzae b (Hib) infections are based on the capsular polysaccharide (PRP) conjugated with a carrier protein. The polysaccharide is a polymer of ribose, ribitol and phosphate. These vaccines are typically presented as plain (ie without adjuvantation) formulations. Although in one case, (Pedvax Hib produce by Merck) a diluent containing aluminium hydroxide is utilised to reconstitute the lyophilised conjugate. Typically the carrier protein is a diphtheria or tetanus toxoid or an outer membrane protein of *N.meningitidis.* Examples of such conjugate vaccine antigens are disclosed in US 4 365 170, US 4 673 574, EP 208 375, EP 477508 and EP 161 188. Bixler et al [Adv.Exp.Med.Biol. (1991) 303:185-190] discloses a composition comprising a CRM197 conjugate of Hib polysaccharide adsorbed onto aluminium phosphate but with no other antigens derived from a pathogen. Siber et al [Vaccine (1995) 13:525-531] describes the development of a guinea pig model to assess immunogenicity of *Haemophilus influenzae* type b capsular polysaccharide conjugate vaccines.

It is desirable to administer such conjugate vaccines with other antigens or vaccines at the same time and this can involve multiple injections. Problems associated with multiple injections include a more complicated administration procedure and a large total injection volume. This is a particularly acute problem when the vaccine is intended for infants.

It has therefore been proposed to produce combination vaccines. EP594950 discloses combination vaccines including unadsorbed Hib polysaccharide conjugates. One well known combination vaccine provides protection against Diphtheria , tetanus and B. pertussis infections. This vaccine comprises a whole cell or an acellular pertussis component which typically consists of two or three antigens - (detoxified PT, FHA and often, but not exclusively 69kDa) although in certain circumstances other B. pertussis antigens may also be present and toxoided diphtheria and tetanus toxins. Such vaccines are often referred to as DTPw or DTPa. Other antigens would desirably be added to such a combination vaccine for the prevention of diseases like hepatitis B or Polio.

It would be desirable to add Hib polysaccharide conjugate vaccines to such a combination. However we have found that simple mixing of the components results in a reduction of antibody titres to the polysaccharide component.

The present inventors have discovered that this reduction can be inhibited if the Hib polysaccharide conjugate antigen is adsorbed on to aluminium phosphate. In contrast, if the antigen is adsorbed on to aluminium hydroxide, there is a complete reduction of antibody titres to the Hib polysaccharide component.

Accordingly the present invention provides a combination vaccine comprising a capsular polysaccharide of *Haemophilus influenzae* B conjugated to a carrier protein **characterised in that** the conjugate antigen is adsorbed onto aluminium phosphate; and an other component selected from the group of antigens which can afford protection against one of diphtheria, tetanus and pertussis disease..

Preferably the carrier protein is either diphtheria or tetanus toxoid, Diphtheria Crm₁₉₇ protein or an outer membrane protein from a bacteria such as *N.meningitidis*.

The polysaccharide conjugate may be prepared by any known coupling technique. For example the polysaccharide can be coupled via a thioether linkage. This conjugation method relies on activation of the polysaccharide with 1-cyano-4-dimethylamino pyridinium tetrafluoroborate (CDAP) to form a cyanate ester. The activated polysaccharide may thus be coupled directly or via a spacer group to an amino group on the carrier protein. Preferably, the cyanate ester is coupled with hexane diamine and the amino-derivatised polysaccharide is conjugated to the carrier protein using heteroligation chemistry involving the formation of the thioether linkage. Such conjugates are described in PCT published application WO93/15760 Uniformed Services University.

The conjugates can also be prepared by direct reductive amination methods as described in US 4365170 (Jennings) and US 4673574 (Anderson). Other methods are described in EP-0-161-188, EP-208375 and EP-0-477508.

A further method involves the coupling of a cyanogen bromide activated polysaccharide derivatised with adipic acid hydrazide (ADH) to the protein carrier by carbodiimide condensation. Such conjugation is described in Chu C. et al Infec Immunity, 1983 245 256.

In a preferred embodiment of the invention the ratio of PRP polysaccharide to carrier protein is reduced from a typical 1:3 to 1:0.3 to 1:2. Such low ratio conjugates are advantageous, since even in an unadjuvanted state, they do not suffer from interference problems.

Particular combination vaccines within the scope of the invention include a DTPa (diphtheria-tetanus-acellular pertussis) -Hib combination vaccine formulation, a DTPa-Hib-Hepatitis B vaccine formulation and an IPV (inactivated polio vaccine) -DTPa-Hib-Hepatitis B vaccine formulation.

The above combinations may optionally include a component which is protective against Hepatitis A.

Suitable components for use in such vaccines are already commercially available and details may be obtained from the World Health Organisation. For example the IPV component may be the Salk inactivated polio vaccine. The Diphtheria, Tetanus and Pertussis vaccine may comprise an acellular product such as Infanrix DTPa (SmithKline Beecham Biologicals) . The component affording protection against Hepatitis A is preferably the product known as 'Havrix' (SmithKline Beecham Biologicals) which is a killed attenuated vaccine derived from the HM-175 strain of HAV [see 'Inactivated Candidate Vaccines for Hepatitis A' by F.E. Andre, A Hepburn and E.D'Hondt, Prog Med. Virol. Vol 37, pages 72-95 (1990) and the product monograph 'Havrix' published by SmithKline Beecham Biologicals (1991)]. The Hepatitis B component may comprise the 'S' antigen as in 'Engerix-B'.

Advantageously the Haemophilus Influenzae B or combination vaccine according to the invention is a paediatric vaccine.

Vaccine preparation is generally described in Vaccine Design - The Subunit and adjuvant approach Ed Powell and Newman; Pellum Press. Encapsulation within liposomes is described, for example, by Fullerton, US Patent 4,235,877. Conjugation of proteins to macromolecules is disclosed, for example, by Likhite, US Patent 4,372,945 and by Armor et al., US Patent 4,474,757.

The amount of conjugate antigen in each vaccine dose is selected as an amount which induces an immunoprotective response without significant, adverse side effects in typical vaccinees. Such amount will vary depending on which specific immunogens are employed. Generally it is expected that each dose will comprise 1-1000µg(ug) of total immunogen, preferably 2-100µg(ug), most preferably 4-40µg(ug). An optimal amount for a particular vaccine can be ascertained by standard studies involving observation of antibody titres and other responses in subjects. Following an initial vaccination, subjects may receive one or two booster injections at about 4 weeks intervals.

In a further aspect according to the invention, there is provided a method of producing a combination vaccine comprising the steps of: conjugating a *Haemophilus influenzae* type B capsular polysaccharide to a carrier protein, adsorbing said conjugate antigen onto aluminium phosphate at pH5-6, and combining said conjugate antigen with an other antigen in liquid form which can afford protection against diphtheria, tetanus or pertussis disease. Preferably adsorption of said conjugate antigen onto aluminum phosphate is carried out at pH5.4. The invention further provides the first medical use of such a vaccine.

The following examples illustrate the invention.

### Example 1

### Vaccine formulation comprising HiB polysaccharide conjugated on Tetanus toxoid adsorbed on to Aluminium phosphate.

### Synthesis of Haemophilus influenzae type B capsular polysaccharide (PRP) Tetanus toxoid (TT) conjugate

### 1.a Cyanogen Bromide Coupling

The covalent binding of PRP and TT is carried out by a coupling chemistry developed at the NIH (Chu C. et al (1983), further studies on the immunogenicity of Haemophilus influenzae type b and pneumococcal type 6A polysaccharide protein conjugates. Infec. Immunity, 245-256). The PRP is activated under controlled conditions by cyanogen bromide and derivatised with an adipic hydrazide spacer.

After derivatisation, the activated polysaccharide (PRP-AH) is purified by diafiltration. The coupling of the two purified components (PRP-AH and TT) is effected by carbodiimide condensation. The conjugate is then purified by ultrafiltration and gel filtration to remove the reagent and unconjugated PRP and TT.

### Synthesis of PRP-TT Conjugates

### 1.b CDAP coupling

30mg of native Hib PRP were dissolved in 6ml 2M NaCl. 225µl (mcl) of CDAP (1 cyano-4-dimethylamino-pyridinum tetrafluoroborate) was added to the polysaccharide solution (from a 100 mg/ml stock solution in acetonitrile). 90 seconds later, 450 µl (mcl) of 0.2 M triethylamine was added. The activation was performed at pH 10.0 during 1 minute on ice and minute at room temperature.

90 mg of tetanus toxoid (initial PS/protein ratio of 1/3) were added to the activated polysaccharide and the coupling reaction was performed at room temperature for 1 hour. Then, the reaction was quenched with 3 ml of 1 M glycine solution, pH 5.0 for 30 minutes at room temperature and overnight at 4°C.

The conjugate was purified by gel filtration on a sephacryl HR 500 column equilibrated in 0.2M NaCl. The carbohydrate and protein content was determined in each fraction. The conjugate was pooled and sterile filtered (membrane Minisart ⊕ 0.222 µm).

### Adsorption on to aluminium phosphate

1.c To 0.15mg of aluminium phosphate was added 12.5 µg (mcg) of the polysaccharide conjugate of example 1(a). This was stirred for two hours the pH is adjusted to 5.1. The mixture was left to stand for one day at room temperature and the adsorbed conjugate then left for a further 9 days at 2 to 8°C. To prepare a freeze dried product the adsorbed product is diluted in lactose (15.75mg) to give a final composition of 25µg (mcg) polysaccharide/ml and 0.4mg Al/ml and the resulting composition was filled into 0.5ml vials and freezed dried.

To prepare a liquid product the adsorbed conjugate is diluted in water for injection with 150mM NaCl and 5mg/ml phenoxy ethanol to give a final composition of 20 µg (mcg) polysaccharide/ml and 0.32 mg Al/ml.
1.d Formulation of a Diphtheria Tetanus and Pertussis (acellular) vaccine with and without hepatitis B was done in accordance to the methods of WO 93/24148 (SmithKline Beecham Biologicals).
1.e Preparation of a 'low ratio' PRP-TT aluminium phosphate pre-adsorbed conjugate.

The conjugate was prepared in an analogous manner to the example of 1a, but with reduced amount of Tetanus used (30µg (mcg), 60µg (mcg)) to give a product with Polysaccharide:Protein ratio of 1:1 or 1:2. The conjugate is then adsorbed on to aluminium phosphate according to the method of example 1c. The final freeze dried preparation contains 12.5µg of conjugate, 0.15mg AlPO₄, 15.75 mg lactose. This is reconstituted in 0.5ml water for injection prior to use at a pH of 0.1 +/- 0.1.

### Example 2: Immunogenicity of PRP-TT conjugate preadsorbed on aluminium phosphate and combined with DTPa or DTPa-HB

The Hib conjugate of example 1a), either plain or pre-adsorbed on Al PO₄ (both vaccines were lyophilized) was mixed with DTPa or DTPa HB no more than 1 hour before injection and the combination was injected in baby rats (1 week of age) by the subcutaneous route at a dose corresponding to 1/20th a human dose (0.5 µg of PRP). The rats were boosted 2 weeks and 4 weeks later and the serum was collected was collected after each immunization to measure anti-PRP antibodies. Controls included the Hib vaccines (adsorbed or not on Al PO₄) reconstituted in saline.

Groups of 10 randomized baby rats (1 week of ago-OFA strain) were immunized 3 times subcutaneously at 0-14-28 days with 1/20th human dose of Hib vaccine, alone or combined with DTPa or DTPa HB (1/20th a human dose). The reconstitution of the lyophilised Hib vaccine with saline or combinations (DTPa or DTPa HB) was done less than 1 hour before immunization.

The rats were bled under anaesthesia at 14-28-42 and 56 days. The anti-PRP antibodies were measured by ELISA in individual sera and the titers were expressed in µg/ml(γ/ml) using a calibrated reference. The GMT was calculated for each group and for each time point. The 95% confidence limits were calculated for the titers obtained after that third immunization.

As shown in table 1, the adsorption of Hib conjugate on A1 PO₄ does not modify its immunogenicity: some anti-PS were produced after the second dose and a good booster effect is shown after the third dose as seen in human babies. The mixing of Hib vaccine with DTPa or DTPa HB reduces by 3 to 8 fold the anti-PRP response and, in the case of DTPa-HB, this decrease is significant. In contrast, the preadsorption of the Hib vaccine on A1 PO₄ restores the anti-PRP response to a level at least equivalent to that obtained with the plain vaccine.

### Conclusion:

The Hib/aluminium phosphate formulation has thus the potential to solve the compatibility problem encountered when mixing Hib with other paediatric combinations.

**Table 1**

| Immunogenicity in a baby rat model of PRP-TT conjugate pre adsorbed on AlP0₄ and combined with DTPa or DTPa-HB | | | | |
|---|---|---|---|---|
| **Vaccine** | **Anti-PRP titre (µg/ml [γ/ml]) at day** | | | |
| | **14 (Post I)** | **28 (Post II)** | **42 (Post III)** | **56 (Post III 30)** |
| None (Nacl 0.9%) | | | | |
| | <0.05 | <0.05 | <0.05 | <0.05 |
| Hib-001 | <0.05 | 0.06 | 12.9 (4-37) | 10.9 (4-31) |
| Hib/AlP04 (Dhib-024) | <0.05 | 1.3 | 11.8 (5-29) | 15.4 (7-35) |
| Hib-001+DTPa (119) | <0.05 | 0.16 | 3.4 (0-28) | 1.4 (0.1-17) |
| Hib/ AlP04 + DTPa (119) | <0.05 | 1.9 | 20.9 (7-59) | 19.7 (9.42) |
| Hib-001+DTPa HB (16705) | <0.05 | 0.14 | 2.8 (1-6) | 3.9 (2-9) |
| Hib/AlP04 +DTPa HB (16705) | <0.05 | 0.47 | 11.4 (5-27) | 18.1 (9-38) |
| Hib/Al(OH₃) | <0.05 | <0.05 | <0.05 | <0.14 |

## Claims

1. A method of producing a combination vaccine comprising the steps of: conjugating a Haemophilus influenzae type B capsular polysaccharide to a carrier protein, adsorbing said conjugate antigen onto aluminium phosphate at pH 5-6, and combining said conjugate antigen with an other antigen in liquid form which can afford protection against diphtheria, tetanus or pertussis disease.

2. The method of claim 1, wherein the conjugate antigen is adsorbed onto aluminium phosphate at about pH 5.4.

3. The method of claim 1 or 2, wherein after adsorbing the conjugate antigen onto aluminium phosphate, the conjugate antigen is left standing for more than 24 hours and then freeze-dried.

4. The method of claims 1-3, wherein the carrier protein is selected from the group consisting of: Diphtheria toxoid, Diphtheria CRM197 protein, Meningococcal outer membrane protein, and tetanus toxoid.

5. The method of claims 1-4, wherein the ratio of the Haemophilus influenzae B capsular polysaccharide to carrier protein in the conjugate antigen is from 1:0.3 to 1:2 (w:w).

6. The method of claims 1-5, wherein the Haemophilus influenzae type B capsular polysaccharide is conjugated to the carrier protein by thioether linkage chemistry or by direct reductive amination chemistry..

7. The method of claims 1-6, wherein the combination vaccine is a DTPa-Hib, DTPa-Hib-Hepatitis B, or IPV-DTPa-Hib-Hepatitis B combination vaccine.

8. The method of claim 7, wherein the combination vaccine comprises antigens consisting of Haemophilus influenzae type B capsular polysaccharide antigen conjugated to a carrier protein, diphtheria toxoid, tetanus toxoid, acellular pertussis antigens, Hepatitis B surface antigen and Inactivated Polio Vaccine.

9. A combination vaccine comprising:
I. a capsular polysaccharide of Haemophilus influenzae B conjugated to a carrier protein **characterised in that** the conjugate antigen is adsorbed onto aluminium phosphate; and
II. an other component selected from the group of antigens which can afford protection against one of diphtheria, tetanus and pertussis disease.

10. The combination vaccine of claim 9 which is a DTPa-Hib, DTPa-Hib-Hepatitis B, or IPV-DTPa-Hib-Hepatitis B combination vaccine.

11. A combination vaccine as claimed in claim 9 or 10 wherein the carrier protein is selected from the group comprising: Diphtheria toxoid, Diphtheria CRM197 protein and Meningococcal outer membrane protein.

12. A combination vaccine as claimed in claim 9 or 10 wherein the carrier protein conjugated to the capsular polysaccharide of Haemophilus influenza B is tetanus toxoid.

13. A combination vaccine as claimed in any one of claims 9-12 wherein the ratio of the Haemophilus influenzae B capsular polysaccharide to carrier protein is from 1:0.3 to 1:2 (w/w).

14. A kit for making a combination vaccine comprising a container of freeze-dried vaccine comprising a capsular polysaccharide of Haemophilus influenzae B conjugated to a carrier protein, wherein the conjugate antigen is adsorbed onto aluminium phosphate, and a second container with a vaccine comprising an other antigen which can afford protection against diphtheria, tetanus or pertussis disease.

15. A combination vaccine as defined in any one of claims 9-14 for use in medicine.

16. The use of a safe and efficacious amount of a combination vaccine as defined in any one of claims 9-14 in the manufacture of a medicament for the treatment of a patient suffering from or susceptible to Haemophilus influenzae B infection.

## Patentansprüche

1. Verfahren zur Herstellung eines Kombinationsimpfstoffs, umfassend die folgenden Schritte: Konjugieren eines Kapselpolysaccharids von Haemophilus influenza-Typ B an ein Trägerprotein, Adsorbieren des konjugierten Antigens auf Aluminiumphosphat bei pH 5 bis 6 und Kombinieren des konjugierten Antigens mit einem anderen Antigen in flüssiger Form, das einen Schutz gegen Diphtherie-, Tetanus- oder Pertussis-Erkrankung gewähren kann.

2. Verfahren gemäss Anspruch 1, worin das konjugierte Antigen auf Aluminiumphosphat bei etwa pH 5,4 adsorbiert wird.

3. Verfahren gemäss Anspruch 1 oder 2, worin das konjugierte Antigen, nachdem das konjugierte Antigen auf Aluminiumphosphat adsorbiert worden ist, für mehr als 24 Stunden stehengelassen und dann gefriergetrocknet wird.

4. Verfahren gemäss Ansprüchen 1 bis 3, worin das Trägerprotein ausgewählt ist aus der Gruppe bestehend aus: Diphtherie-Toxoid, Diphtherie-CRM197-Protein, Meningokokken-Aussenmembranprotein und Tetanus-Toxoid.

5. Verfahren gemäss Ansprüchen 1 bis 4, worin das Verhältnis von Kapselpolysaccharid von Haemophilus influenza B zu Trägerprotein im konjugierten Antigen von 1:0,3 bis 1:2 (G/G) ist.

6. Verfahren gemäss Ansprüchen 1 bis 5, worin das Kapselpolysaccharid von Haemophilus influenza-Typ B an das Trägerprotein durch Thioether-Verknüpfungschemie oder durch direkte reduktive Aminierungschemie konjugiert wird.

7. Verfahren gemäss Ansprüchen 1 bis 6, worin der Kombinationsimpfstoff ein DTPa-Hib-, DTPa-Hib-Hepatitis B- oder IPV-DTPa-Hib-Hepatitis B-Kombinationsimpfstoff ist.

8. Verfahren gemäss Anspruch 7, worin der Kombinationsimpfstoff Antigene umfasst, bestehend aus Kapselpolysaccharid-Antigen von Haemophilus influenza-Typ B, konjugiert an ein Trägerprotein, Diphtherie-Toxoid, Tetanus-Toxoid, azelluläre Pertussis-Antigene, Hepatitis B-Oberflächenantigen und inaktiviertem Polioimpfstoff.

9. Kombinationsimpfstoff, umfassend:
(I) Kapselpolysaccharid von Haemophilus influenza B, konjugiert an ein Trägerprotein, **dadurch gekennzeichnet, dass** das konjugierte Antigen auf Aluminiumphosphat adsorbiert ist; und
(II) einen anderen Bestandteil, ausgewählt aus der Gruppe von Antigenen, die einen Schutz gegen eines von Diphtherie-, Tetanus- und Pertussis-Erkrankung gewähren können.

10. Kombinationsimpfstoff gemäss Anspruch 9, der ein DTPa-Hib-, DTPa-Hib-Hepatitis B- oder IPV-DTPa-Hib-Hepatitis B-Kombinationsimpfstoff ist.

11. Kombinationsimpfstoff gemäss Anspruch 9 oder 10, worin das Trägerprotein ausgewählt ist aus der Gruppe umfassend: Diphtherie-Toxoid, Diphtherie-CRM197-Protein und Meningokokken-Aussenmembranprotein.

12. Kombinationsimpfstoff gemäss Anspruch 9 oder 10, worin das an das Kapselpolysaccharid von Haemophilus influenza B konjugierte Trägerprotein Tetanus-Toxoid ist.

13. Kombinationsimpfstoff gemäss irgendeinem der Ansprüche 9 bis 12, worin das Verhältnis von Kapselpolysaccharid von Haemophilus influenza B zu Trägerprotein von 1:0,3 bis 1:2 (G/G) ist.

14. Kit zur Herstellung eines Kombinationsimpfstoffs, umfassend einen Behälter mit einem gefriergetrockneten Impfstoff, umfassend ein Kapselpolysaccharid von Haemophilus influenza B, konjugiert an ein Trägerprotein, worin das konjugierte Antigen auf Aluminiumphosphat adsorbiert ist, und einen zweiten Behälter mit einem Impfstoff, umfassend ein anderes Antigen, das einen Schutz gegen Diphtherie-, Tetanus- oder Pertussis-Erkrankung gewähren kann.

15. Kombinationsimpfstoff gemäss irgendeinem der Ansprüche 9 bis 14 zur Verwendung in der Medizin.

16. Verwendung einer sicheren und wirksamen Menge eines Kombinationsimpfstoffs gemäss irgendeinem der Ansprüche 9 bis 14 in der Herstellung eines Medikaments zur Behandlung eines Patienten, der unter einer Haemophilus influenza B-Infektion leidet oder dafür empfänglich ist.

## Revendications

1. Procédé de production d'un polyvaccin, comprenant les étapes suivantes : la conjugaison d'un polysaccharide capsulaire d'Haemophilus influenzae de type B à une protéine support, l'adsorption dudit antigène conjugué sur du phosphate d'aluminium à pH 5-6 et la combinaison dudit antigène conjugué avec un autre antigène dans une forme liquide pouvant fournir une protection contre la diphtérie, le tétanos et la coqueluche.

2. Procédé selon la revendication 1, dans lequel l'antigène conjugué est adsorbé sur du phosphate d'aluminium à un pH d'environ 5,4.

3. Procédé selon la revendication 1 ou 2, dans lequel après l'adsorption de l'antigène conjugué sur le phosphate d'aluminium, l'antigène conjugué est laissé au repos pendant plus de 24 heures puis lyophilisé.

4. Procédé selon les revendications 1 à 3, dans lequel la protéine support est choisie dans le groupe constitué par l'anatoxine diphtérique, la protéine CRM197 de la diphtérie, la protéine membranaire externe de méningocoque et l'anatoxine tétanique.

5. Procédé selon les revendications 1 à 4, dans lequel le rapport entre le polysaccharide capsulaire d'Haemophilus influenzae B et la protéine support dans l'antigène conjugué est de 1 : 0,3 à 1 : 2 (en poids).

6. Procédé selon les revendications 1 à 5, dans lequel le polysaccharide capsulaire d'Haemophilus influenzae de type B est conjugué à la protéine support par une chimie de formation de liaison thioéther ou par une chimie d'amination réductrice directe.

7. Procédé selon les revendications 1 à 6, dans lequel le polyvaccin est un polyvaccin DTPa-Hib, DTPa-Hib-hépatite B ou IPV-DTPa-Hib-hépatite B.

8. Procédé selon la revendication 7, dans lequel le polyvaccin comprend des antigènes consistant en l'antigène polysaccharidique capsulaire d'Haemophilus influenzae de type B conjugué à une protéine support, l'anatoxine diphtérique, l'anatoxine tétanique, des antigènes acellulaires de coqueluche, l'antigène de surface de l'hépatite B et le vaccin antipoliomyélitique inactivé.

9. Polyvaccin comprenant :
I. un polysaccharide capsulaire d'Haemophilus influenzae B conjugué à une protéine support, **caractérisé en ce que** l'antigène conjugué est adsorbé sur du phosphate d'aluminium ; et
II. un autre composant choisi dans le groupe des antigènes pouvant fournir une protection contre une maladie sélectionnée parmi la diphtérie, le tétanos et la coqueluche.

10. Polyvaccin selon la revendication 9, qui est un polyvaccin DTPa-Hib, DTPa-Hib-hépatite B ou IPV-DTPa-Hib-hépatite B.

11. Polyvaccin selon la revendication 9 ou 10, dans lequel la protéine support est choisie dans le groupe comprenant : l'anatoxine diphtérique, la protéine CRM197 de la diphtérie et la protéine membranaire externe de méningocoque.

12. Polyvaccin selon la revendication 9 ou 10, dans lequel la protéine support conjuguée au polysaccharide capsulaire d'Haemophilus influenzae B est l'anatoxine tétanique.

13. Polyvaccin selon l'une quelconque des revendications 9 à 12, dans lequel le rapport entre le polysaccharide capsulaire d'Haemophilus influenzae B et la protéine support est de 1 : 0,3 à 1 : 2 (en poids).

14. Kit permettant de préparer un polyvaccin, comprenant un récipient contenant un vaccin lyophilisé comprenant un polysaccharide capsulaire d'Haemophilus influenzae B conjugué à une protéine support, dans lequel l'antigène conjugué est adsorbé sur du phosphate d'aluminium, et un second récipient contenant un vaccin comprenant un autre antigène qui peut fournir une protection contre la diphtérie, le tétanos ou la coqueluche.

15. Polyvaccin selon l'une quelconque des revendications 9 à 14, pour un usage en médecine.

16. Utilisation d'une quantité sûre et efficace d'un polyvaccin selon l'une quelconque des revendications 9 à 14, dans la fabrication d'un médicament destiné au traitement d'un patient sensible à ou souffrant d'une infection par Haemophilus influenzae B.
